Europäisches Patentamt

European Patent Office

Office européen des brevets

(11) Publication number: **0 254 384**
**A2**

(19)

# (12) EUROPEAN PATENT APPLICATION

(21) Application number: 87302688.4

(22) Date of filing: 27.03.87

(51) Int. Cl.⁴: **C12P 21/00** , C12N 5/00 , G01N 33/577 , G01N 33/558 , G01N 33/569

(30) Priority: 27.03.86 US 844677
27.03.86 US 844881

(43) Date of publication of application:
**27.01.88 Bulletin 88/04**

(84) Designated Contracting States:
**AT BE CH DE FR GB IT LI LU NL SE**

(71) Applicant: **CETUS CORPORATION**
**1400 Fifty-Third Street**
**Emeryville California 94608(US)**

(72) Inventor: **Coates, Stephen R.**
**14 Via Floreado**
**Orinda, California 94563(US)**
Inventor: **Saeed, Fawzia A.**
**2750 Dwight Way**
**Berkeley, California, 94704(US)**
Inventor: **Madsen, Randall D.**
**765 Duke Circle,**
**Pleasant Hill, California, 94523(US)**

(74) Representative: **Bizley, Richard Edward et al**
**BOULT, WADE & TENNANT 27 Furnival Street**
**London EC4A 1PQ(GB)**

(54) **Direct and simultaneous assays for antigens in clinical specimens by monoclonal antibody immunoblot, antibodies for use therein and corresponding hybridomas.**

(57) This invention relates to a diagnostic method of employing a monoclonal antibody in a Western immunoblot assay for detecting antigens in clinical specimens. This invention further provides a method of simultaneously detecting a variety of antigens in a single Western immunoblot assay by incorporating in said assay monoclonal antibodies to each antigen to be detected. Said method presents a differential diagnosis procedure employing a panel of monoclonal antibodies to antigens of various symptomatically related etiologic agents.

This invention further relates to a murine IgG1 monoclonal antibody which binds to a protease sensitive, periodate insensitive epitope on Mycoplasma pneumoniae membrane polypeptide with an approximate molecular weight of 43K. It also specifically relates to a diagnostic method for detecting M. pneumoniae with said monoclonal antibody in clinical specimens.

EP 0 254 384 A2

FIG. I

# DIRECT AND SIMULTANEOUS ASSAYS FOR ANTIGENS IN CLINICAL SPECIMENS BY MONOCLONAL ANTIBODY IMMUNOBLOT, ANTIBODIES FOR USE THEREIN AND CORRESPONDING HYBRIDOMAS

This invention extends the diagnostic use of immunoblot tests, known as Westerns, by incorporating therein monoclonal antibodies to different infectious disease etiologic agents. This invention provides for the simultaneous detection of a variety of antigens from microorganisms in one assay. Therefore, this invention provides a greatly improved procedure for differential diagnosis of etiologic agents from a single clinical specimen, in that, such a differential diagnosis can be made in a single assay rather than in multiple assays.

This invention also relates to a murine IgG1 monoclonal antibody which binds to a protease sensitive, periodate insensitive epitope on a 43 kilodalton (K) molecular weight Mycoplasma pneumoniae membrane polypeptide. The invention further specifically relates to a diagnostic method of detecting M. pneumoniae employing said monoclonal antibody in an immunoblot analysis (Western).

Towbin et al. (refs. 11, 14, 16) and Bittner et al (ref. 15) provide general background on the Western immunoblot assay technique. [Reference citations refer to those listed after the Examples herein.] Towbin et al.'s U.S. Patent (ref. 14) and U.K. Patent No. 0050424 to Erlich et al. describe the diagnostic use of Western immunoblots to detect antigens with polyclonal antibodies. Neither the Erlich et al. UK patent nor the Towbin et al. U.S. patent describes nor teaches the use of the Western immunoblot for the simultaneous assay of different antigens; nor do either describe the incorporation of monoclonal antibodies in Western immunoblots.

McMichael et al. (ref. 17) describes an immunoblot (Western) assay to detect a hepatitis B surface antigen using polyclonal antibodies. Gallo et al. (U.S. Patent No. 4,520,113) detected antibodies to the HTLV-III virus in sera of patients with AIDS and pre-AIDS (Acquired Immune Deficiency Syndrome) by a strip radioimmunoassay (RIA) based on the Western Blot technique or by an enzyme-linked immunosorbent assay (ELISA). Gallo et al. employed an immunoblot (Western) assay to eliminate non-specific bindings in the ELISA tests which would otherwise be recorded as false positive test results. The diagnostic method of this invention is distinguishable from such methods in that the immunoblot (Western) assay is employed to detect directly an antigen in a clinical specimen and by its use of monoclonal, rather than a polyclonal antibodies.

The use of monoclonal antibodies provides the advantage of being able to test clinical specimens containing numerous polypeptides and various antigens. Employing polyclonal antibodies in a Western immunoblot to test such a specimen results in excessive background, that is, non-specific binding of polyclonal antibodies to non-specific specimen polypeptides and multiple pathogen-specific antigens. Such cross-reactivity with polyclonal antibodies results in ambiguous test results that would be too unclear for accurate diagnosis. Whereas using monoclonal antibody against a specific epitope in a Western immunoblot results in a clear band, easily distinguishable from background staining. Thus, a clear improvement over the prior art results from employing monoclonal antibodies in a diagnostic Western immunoblot in that clinical specimens, rather than purified, partially purified or cultured infectious disease organisms, can be employed.

Further, an immunoblot assay incorporating monoclonal antibody is efficient. Such an assay as described below can not only be performed directly on clinical specimens, but also can be completed within five hours when precast minigels (100 mm × 80 mm) are employed.

Simultaneous immunoassays have been described for radioimmunoassays and enzyme immunoassays. M. Kuriyama et al. (ref. 21) describe the advantage of using a combination rest of tissue-specific markers to detect the presence of two proteins of human prostate-specific origin, namely, prostatic acid phosphatase and prostate antigen. Kuriyama et al., however, did not detect the two antigens by a simultaneous method but rather combined the results of the separate measurements of each antigen.

Mitsuma et al. (1972) (ref. 22), Ljunggren et al. (ref. 23) and Haynes et al. (ref. 24) used indirect competitive radioimmunoassay to measure more than one component in a single tube by labeling antigens with two different iodine isotopes, including $^{131}I$ which has a half life of only 8 days. Vihko et al. (ref. 25) disclose immobilizing two types of antibodies on different sections of small test tubes and joining them to produce a single multicomponent tube. The haptens are labeled with $^{125}I$, which is detected by indirect radioimmunoassay. This method represents a relatively complex procedure involving separation of the solid phase carrier.

U.S. Patent No. 4,315,907 to Fridlender et al. and Gehle et al. (ref. 29) describe a specific binding assay to determine multiple antigens employing solid-phase binding agents corresponding to each antigen which are differentially separable, as well as labeled binding agents for each antigen. Each solid-phase bound species is separated from all the other species after incubation.

Blake et al., (ref. 28) describe the simultaneous enzyme immunoassay of two thyroid hormones which has advantages over radioisotopes in that it is stable and allows for facile differential measurement. In this assay a mixture of two conjugates labeled with two different enzymes is used to detect both antigens simultaneously in a fluid. The enzymes form products easily distinguishable from each other by adsorption spectrophotometry. Blake et al. used two assay compounds, i.e., two antibodies immobilized on two separate solid carriers, to detect both antigens, rather than a single assay compound.

South African Patent No. 85/2574 granted November 26, 1986 discloses an improved process for simultaneously detecting more than one antigen, most preferably prostate antigen and prostatic acid phosphatase. A single immunometric assay material is used to detect all of the antigens in the test fluid. Thus, the optimization of amounts of conjugates to be used in the assay mixture for the measurement is simplified. Furthermore, in such assay the measurement is direct rather than an indirect competitive immunoassay.

However, all of the above simultaneous immunoassays described for radioimmunoassays and enzyme immunoassays require different labels (either enzymatic or radioactive) for each specific antibody detected. Further, such assays are generally limited to detecting two, and at most, potentially three antigens. A great advantage of the instant invention's simultaneous detection system is, not only the time efficiency provided and the capability of directly testing clinical specimens, but also the capability of the system of simultaneously assaying for a variety of antigens.

An exemplary monoclonal antibody which can be used in the simultaneous detection system specifically for differntial diagnosis of pneumonia, or alone to detect directly Mycoplasma pneumoniae (M. pneumoniae) is a murine IgG1 which binds to a protease sensitive, periodate insensitive epitope on a 43K M. pneumoniae membrane polypeptide. Such epitope is present in all clincial M. pneumoniae isolates tested, but was not found in normal flora mycoplasma species or other organisms found in the human respiratory tract.

Mycoplasma pneumoniae is a human respiratory tract pathogen commonly causing tracheobronchitis, atypical pneumonia and pharyngitis. [Cassel, G.H. and B.C. Cole. 1981. Mycoplasma as agents of human disease. N. Engl. J. Med. 304: 80-89; Clyde, W.A. Jr. 1979. Mycoplasma pneumoniae infections in man. p.275-306. In J.G. Tully and R.F. Whitcomb (eds.) The Mycoplasmas. Vol. 2. Academic Press, New York.] M. pneumoniae has also been implicated in extrapulmonary complications including neurologic disease, erythema multiforme, bullous myringitis, neurolytic anemia, myocarditis and pericarditis. [Levine, D.P., and A.M. Levner. 1978. The clinical spectrum of Mycoplasma pneumoniae infections. Med. Clin. North Am. 62:961-978; Murray, H.W., H. Masur, L.B. Senterfit, and R.B. Roberts. 1975. The protean manifestations of Mycoplasma pneumoniae infections in adults. Am J. Med. 58:229-242; and Ponka, A. 1979. The occurrence and clinical picture of serologically verified Mycoplasma pneumoniae infections with emphasis on central nervous system, cardiac and joint manifestations. Ann. Clin. Res. Suppl 24:1-60] While the incidence rate is highest among children and young adults [Foy, H.M., G.F Kenny, M.F. Conney, and I.D. Allen. 1979. Long-term epidemiology of infections with Mycoplasma pneumoniae . J. Infect Dis. 139:681-687; and Foy, H.M., and I.D. Allen. 1982. Frequency of Mycoplasma pneumoniae pneumonia. Lancet xx:392], M. pneumoniae infections have been observed in all age groups (Murray, H.W. et al., supra; and Ponka, A., supra). Erythromycin or tetracycline therapy has been shown to be efficacious in M. pneumoniae infections. [Dean, N.L. 1981. Mycoplasmal pneumoniae in the community hospital. Clin. Chest. Med. 2:121-131 (ref. 1); and Shames, J.M., R.B. George, and W.B. Holliday. 1970. Comparison of antibiotics in the treatment of mycoplasmal pneumonia. Arch. Intern. Med. 125:680-684]

Clinical symptoms may support the diagnosis, but they are not distinct from those resulting from viral, group A streptococcal infections or other bacterial respiratory infections. (Clyde, W.A. Jr., supra) Currently, definitive diagnosis is demonstrated by culture isolation of the organism or a four-fold rise in specific antibody between acute and convalescent paired sera. (Clyde, id.) Results may require 1-3 weeks and, although yielding retrospective information, are not helpful in guiding initial therapy. In addition, for many specimens, culture results cannot be obtained because of media overgrowth by faster growing normal flora bacteria. (Tully, J.G., R.F. Whitcomb and T.F. Clark. 1977. Pathogenic mycoplasmas: cultivation and vertebrate pathogenicity of a new spiroplasma. Science 195: 892-894)

Current serological diagnostic procedures for M. pneumoniae infections include testing for cold hemagglutinins (IgM anti-Ia antibodies) [Griffin, An. Intern. Med. 70:701 (1967)] and M. pneumoniae specific antibodies by complement fixation [Kenney et al., J. Immunol. 95:19 (1965)], enzyme immunoassay [Busolo et al., J. Clin. Micro. 12:29 (1980); Dussaix et al., J. Clin. Path. 36:228 (1983)] and indirect fluorescent immunoassay [Umetsu et al. Tohoku J. Exp. Med. 116:213 (1975)]. Isolation of M. pneumoniae from sputum or throat swabs provides definitive information but requires seven days to three weeks for results. Since current diagnostic methods cannot be performed quickly enough to guide therapy in these infections, a rapid diagnostic test would be highly useful.

Direct detection of M. pneumoniae antigens in clinical specimens has been attempted using polyclonal antibody by counterimmune-electrophoresis [Wiernik et al., Scand. J. Inf. Dis. 10:173-176 (1978)] and fluorescent antibody assays [Bayer et al., Anal. of Int. Med. 94:15 (1980); Hers, Am. Rev. Resp. Dis. 88:316 (1983) (ref. 5)], but with limited success. Enzyme immunoassays have been reported to detect M. pneumoniae antigens [**Helbig, J.H., and W. Witzleb.**1984. Enzyme-linked immunosorbent assay (ELISA) Zum Antigennachweis von Mycoplasma pneumoniae Z. ges. Hyg. 30:106-107; and **Kist, M., E. Jacobs, and W. Bredt.** 1982. Release of Mycoplasma pneumoniae substances after phagocytosis by guinea pig alveolar macrophages. Infect. Immun. 36: 357-362], but have not been investigated with clinical specimens.

Although monoclonal antibodies to M. pneumoniae whole organism [**Buck, D.W., R.H. Kennett, and G. McGarrity.** 1982. Monoclonal antibodies specific for cell culture mycoplasmas. In Vitro. 18:377-381], surface adhesion protein, P1, [**Feldner et al.** 1982 Nature 298:765-767; **Hu, et al.** 1981. Identification of immunogens of Mycoplasma pneumoniae by protein blotting. Biochem. Biophys. Res. Commun. 103:1363-1370], and lipid antigens [**Morrison-Plummer, J., D.H. Jones, and J.B. Baseman.** 1983. An ELISA to detect monoclonal antibodies specific for lipid determinants of Mycoplasma pneumoniae. J. Immunol. Meth. 64: 165-178] have been previously described, their diagnostic use has not been reported.

The 43K polypeptide appears to be one of several dozen membrane polypeptides which comprise over 50% of total dry weight of mycoplasma membranes [**Razin, S.** 1979. Membrane proteins, p.289-322. In M.F. Barile and S. Razin (eds.), The Mycoplasmas, Vol. 1, Academic Press, New York] and is one of the antigenic components of the organism as estimated by immunoblot patterns that the instant inventors and other investigators [**Hu et al.**, Biochem. Biophys. Res. Commun. 103:1363-1370 (1981); and **Kenny, G.E., and F.D. Cartwright.** 1984. Immunoblotting for determination of the antigenic specificities of antibodies to the Mycoplasmatales. Isr. J. Med. Sci. 20:908-911] have observed. However, none of such other investigators have designated or described any antigen as an approximately 43K antigen. Kahane et al., [Inf. and Immun., Vol. 50, No. 3:944-946 (1985)] do designate a 41K and 42K antigen, but do not suggest employing such antigens or monoclonal antibodies to such antigens diagnostically.

## Description of the Invention

This invention in a broad sense relates to the use of monoclonal antibodies in Western immunoblot assays to detect defined antigens in clinical specimens. Such an assay is herein exemplified by the use of a murine IgG1 monoclonal antibody which binds to a specific Mycoplasma pneumoniae membrane polypeptide which is defined as having an appropriate molecular weight of 43K and a protease sensitive, periodate insensitive epitope.

This invention further provides a method of detecting simultaneously a variety of antigens in a single Western immunoblot assay by incorporating in said assay monoclonal antibodies to each antigen to be detected. Because of the physical dimensions of Western immunoblot gels, presently commonly in use, the preferable number of antigens to be tested would be from 2 to 10, and most preferably 2 to 8 antigens. Depending on the molecular weights of the antigens for which the monoclonal antibodies are directed, gels would be selected appropriately to provide adequate spatial separation for distinguishing bands in the test results. Ones of ordinary skill in the art would be able to select the appropriate gradient gel or percent gel in accordance with the particular panel of monoclonal antibodies to be employed in the assay. As a rule of thumb, for a series of monoclonal antibodies directed toward lower molecular weight antigens a higher percent gel would be employed. For example, a 10% acrylamide gel may be insufficient to resolve antigens of molecular weights below 25K. In such a situation, a higher percent gel, as, for example, a 12% or 14% gel may need to be employed.

This invention also relates to a differential diagnostic method. Such a method would comprise the steps of

(a) selecting an appropriate panel of monoclonal antibodies, each of which is diagnostic for one possible etiologic agent of clinical symptoms displayed by the patient under diagnosis;

4

(b) employing said monoclonal antibodies in a Western immunoblot assay to test a single clinical specimen of said patient; and

(c) diagnosing or eliminating the etiologic agents tested for in such immunoblot by examining the appearance or non-appearance of the specific bands in the test results for correspondence to the molecular weights of target antigens from said etiologic agents.

Further, this invention relates to a specific differential diagnostic method for detecting the presence of Mycoplasma pneumoniae, Legionella pneumophila and/or Chlamydia (psittaci and trachomatis).

This invention also relates to diagnostic kits comprising monoclonal antibodies directed toward antigens of specific etiologic agents for use in a Western immunoblots. Said diagnostic kits can also comprise an indicator system and any other components that one skilled in the art could use to perform the assays of the instant invention.

Also this invention relates to the use of in a Western immunoblot of a monoclonal antibody to either a 5K genus-specific antigen diagnostic for Chlamydia, that is, for either the species trachomatis or psittaci thereof, or to a 29K diagnostic antigen for Legionella pneumophila. Both molecular weights for said antigens are approximated by methods known to those skilled in art..

An alternative or improved enzyme detection system could increase the sensitivity of the assay system of this invention compared to culture. Although in the examples below, an enzyme detection system is employed, specifically biotinylated anti-mouse IgG and avidin-biotin-peroxidase complex with 3,3'-diaminobenzidine as chromogen, ones skilled in the art would realize that any other enzyme detection system would also be effective. Further, fluorescent-or radio-labelling can be employed. Also, the species-specific monoclonal antibodies employed in this invention could also be labelled themselves for a direct detection system. Further, labelled Staphylococcus protein A could also be used for a detection system.

This invention further relates to a murine IgG1 monoclonal antibody which binds to a protease sensitive, periodate insensitive epitope on a 43 kilodalton (K) molecular weight Mycoplasma pneumoniae membrane polypeptide. It is understood by those in the art that the designation of a numerical molecular weight to a polypeptide is an approximation which encompasses those molecular weights within ± 5% of the stated figure. Upon immunoblot analysis (Western), the 43K polypeptide appeared as the major antigenic component of the M. pneumoniae. Herein is described the M. pneumoniae species-specific MAb and its reactivity in enzyme-linked immunoassays (EIA), immunostaining and immunoblot assays. The monoclonal antibody reacted with 29 different clinical isolates of M. pneumoniae, but did not react with normal flora mycoplasma species or 18 other microorganisms that can also inhabit the normal or diseased human respiratory tract.

This invention also relates to a diagnostic method of detecting M. pneumoniae employing said monoclonal antibody in an immunoblot analysis (Western). Using the M. pneumoniae species-specific monoclonal antibody in an immunoblot assay, sputum specimens from patients with atypical pneumonia and throat swabs from patients with pharyngitis were tested for the presence of the 43K molecular weight polypeptide. The 43K polypeptide was detectable in 3 out of 3 M. pneumoniae culture positive sputum specimens from pneumonia patients. Bound MAb was detected using biotinylated anti-mouse IgG and avidin-biotin-peroxidase complex with 3,3'-diaminobenzidine as chromogen. This assay was shown to detect specific 43,000 MW polypeptide in spiked sputum samples containing 8 ng of M. pneumoniae Lowry protein. Sputum specimens obtained from atypical pneumonia patients having cold-agglutinin titer ≥ 1:128 were subsequently assayed for M. pneumoniae specific polypeptide. These sputums were untestable by conventional mycoplasma culture methods due to bacterial overgrowth. This M. pneumoniae specific 43,000 MW polypeptide was detected in 10 of 24 atypical pneumonia specimens, but not in 10 sputums from normal individuals. Use of rabbit polyclonal anti-M. pneumoniae antibody in this immunoblot assay showed binding to nonspecific sputum proteins which obscured the detection of M. pneumoniae specific polypeptides. This immunoblot assay can be performed in 5 hours and has been shown to be a sensitive and useful method to detect M. pneumoniae antigen in sputum specimens. Further, the 43K polypeptide was also detectable in 29 out of 30 M. pneumoniae culture-confirmed throat swabs from patients with pharyngitis. The 43K polypeptide was, however, not detected in 3 sputum specimens which had been culture-confirmed for Legionella pneumophila, nor in 10 sputum specimens from normal individuals, that is, with no symptoms of respiratory disease, nor in 25 throat swabs from normal, respiratory healthy individuals.

Attempting to detect M. pneumoniae specific antigens directly in a sputum specimen by immunoassay presents several difficult technical problems. Liquification of the sample and solubilization of antigen must be addressed. Additional problems can be encountered with nonspecific false positive reactions caused by the adhesive nature of the matrix of this type of specimen.

5

The MAb immunoblot assay of this invention is very capable of directly addressing these difficulties. The use of SDS and DTT treatment prior to electrophoresis completely liquifies the sample and solubilizes the M. pneumoniae polypeptides. In addition, the 43K polypeptide is spatially separated from any specimen proteins potentially causing nonspecific false positive reactions. One additional advantage of this assay is its ability to detect antigen already complexed with antibody [Burnette, W.N. 1981. "Western Blotting": Electrophoretic transfer of proteins from sodium dodecyl sulfate-polyacrylamide gels to unmodified nitrocellulose and radiographic detection with antibody and radioiodinated protein A. Anal. Biochem. 112: 195-203]. Antigen in the form of immune complexes cannot be detected by conventional immunoassays [Mizutani, H., and H. Mizutani. 1984. Circulating immune complexes in patients with mycoplasmal pneumonia. Am. Rev. Respir. Dis. 130:627-629] and may not be detected by culture. This assay is also not affected by protein-A binding of antibody by staphylococcus found in the specimen which causes false positive results in other immunoassays. [Gosting, L.H. K. Cabrian, J.C. Sturge, and L.C. Goldstein. 1984. Identification of a species - specific antigen in Legionella pneumophila by a monoclonal antibody. J. Clin. Microbial 20:1031-1035]

This invention further relates to a diagnostic test kit specific for the detection of M. pneumoniae infections. Such kit would principally contain the species-specific monoclonal antibody of this invention which recognizes the approximately 43K molecular weight M. pneumoniae membrane polypeptide.

The assay techniques of this invention are efficient in providing results within five hours when precast minigels (80 mm × 100 mm) are employed. With the use of commercially available precast gels, this system is not much more complicated than a standard enzyme immunoassay. It definitely provides an alternative assay method for samples that can not be tested by conventional culture due to the overgrowth of normal flora organisms. Further, the immunoblot assays of this invention can be performed directly on clinical specimens.

Figure Legends

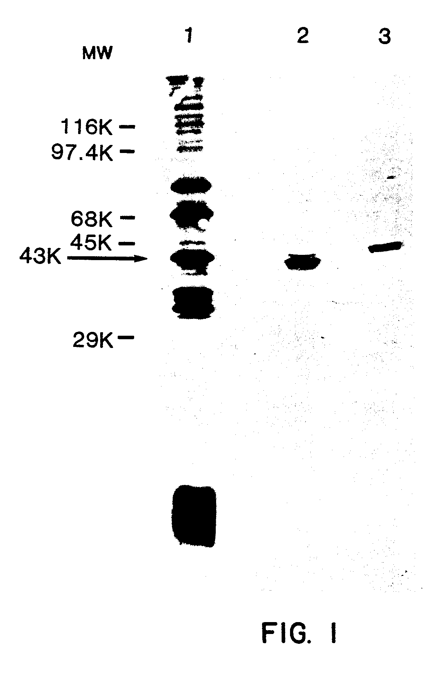

Fig. 1. Immunoblot with rabbit polyclonal and MAb MP16C9. SDS-PAGE gradient gels of whole M. pneumoniae organism or purified membrane preparations were electrophoretically transferred to nitrocellulose paper and reacted with 1:200 dilution of rabbit polyclonal anti-M. pneumoniae serum or 1:5000 dilution of MAb MP16C9 ascites.

Lane 1: whole M. pneumoniae organism probed with rabbit polyclonal;
Lane 2: whole M. pneumoniae organism probed with MAb MP16C9; and
Lane 3: purified M. pneumoniae membrane preparation probed with MAb MP16C9. Bound antibody was detected with antirabbit or antimouse horseradish peroxidase-conjugated antiserum. 3, 3'-diaminobenzidine tetrahydrochloride with nickel chloride was the chromogen used.

Fig. 2. Mycoplasma species profiles on SDS-PAGE gels. This 6-20% acrylamide slab gradient gel was silver stained. Lanes:
1, M. pneumoniae;
2, M. orale;
3, M. salivarium;
4, M. buccale;
5, M. faucium;
6, M. hominis;
7, M. lipophilum;
8, M. fermentans.

Fig. 3. Immunoblot analysis of Mycoplasma species with MAb MP16C9. SDS-PAGE gels of various whole Mycoplasma species organisms were electrophoretically transferred to nitrocellulose paper and probed with a 1:5000 dilution of MAb Mp16C9 ascites. Lanes:
1, M. pneumoniae;
2, M. salivarium;
3, M. orale;
4, M. lipophilum;
5, M. hominis;
6, M. faucium;
7, M. fermentans; and
8, M. buccale.

Fig. 4. Immunostaining of M . pneumoniae organisms with MAb MP169. Total magnification 1000×.

Fig. 5. Immunoblot analysis for 43,000 MW M. pneumoniae polypeptide in sputum specimens from atypical pneumonia patients.

Lane 1 - M. pneumoniae in PBS serving as positive control for location of 43,000 MW polypeptide;

Lanes 2 through 13 - sputum specimens.

Single bands comigrating with M.pneumoniae 43,000 MW polypeptide occur in sputums located in lanes 3,5,6,7,10 and 11.

Fig. 6. Sensitivity of MAb immunoblot assay for M. pneumoniae 43,000 MW polypeptide in spiked sputum.

Lane 1 - prestained molecular weight markers;

lane 2 - 1600 ng/ml of spiked M. - pneumoniae;

lane 3 - 800 ng/ml;

lane 4 - 400 mg/ml;

lane 5 - 200 ng/ml;

lane 6 - 100 ng/ml;

lane 7 - 50 ng/ml;

lane 8 - 25 ng/ml;

lane 9 - 12.5 ng/ml;

lane 10 - unspiked sputum.

43,000 MW immunoblot bands were detected in lanes 2-6.

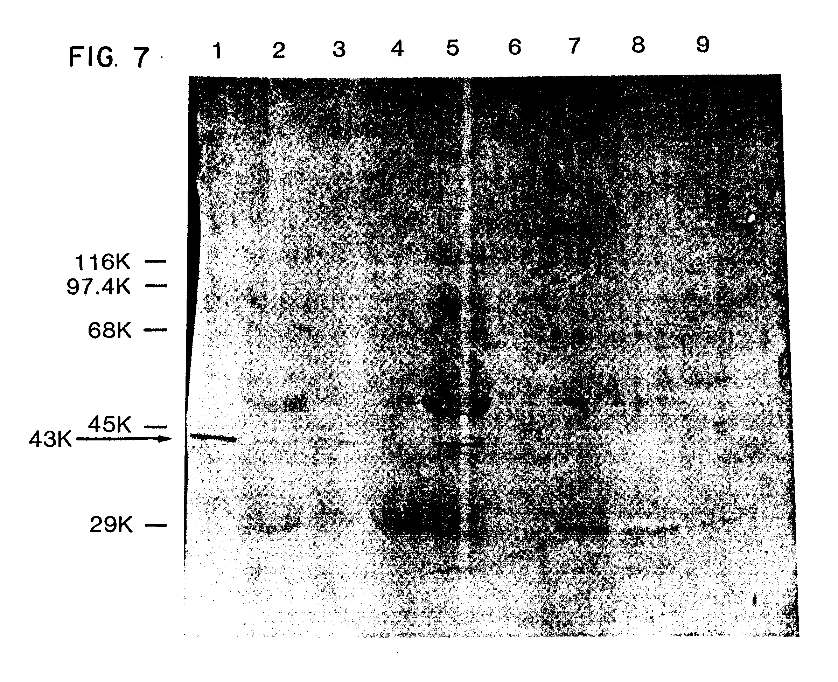

Fig. 7. MAb immunoblot analysis of M. pneumoniae culture-confirmed sputums and nonculturable atypical pneumonia sputums.

Lane 1 - prestained molecular weight markers;

lane 2 - M. pneumoniae 43,000 MW positive control;

lanes 3, 4, 5 - culture-confirmed sputums;

lanes 5 through 10 - atypical pneumonia sputums.

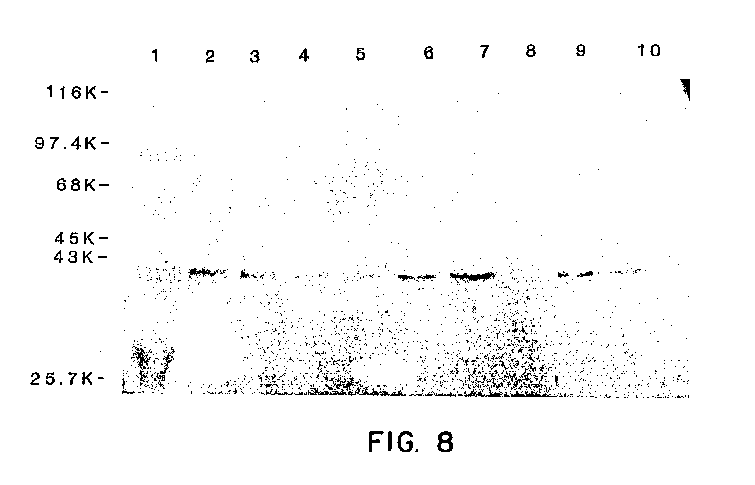

Fig. 8. Immunoblot analysis of M. pneumoniae culture-confirmed throat swabs from patients with pharyngitis.

Lane 1 - prestained molecular-weight markers;

lane 2 - M. pneumoniae 43,000 MW positive control;

lanes 3 through 10 - throat swabs from pharyngitis patients.

Immunoblot bands comigrating with M. pneumoniae 43,000 MW polypeptide were detected in lanes 2-7, 9 and 10.

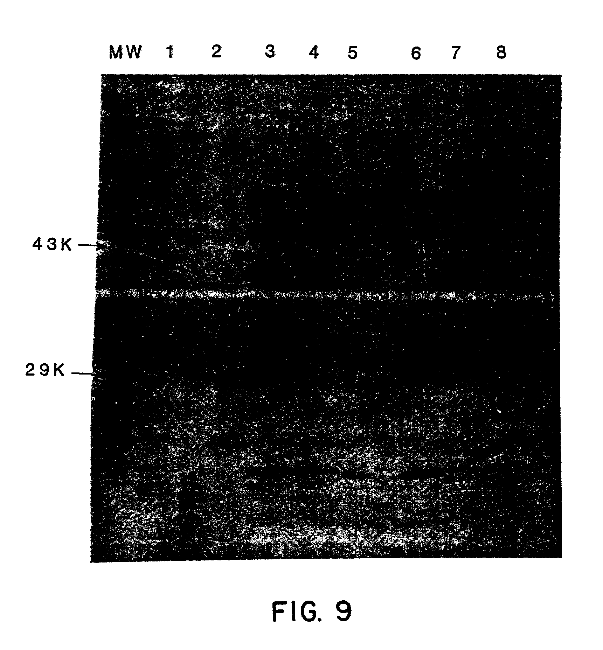

Fig. 9. Illustrates results from testing of clinical specimens.

Lane 1: Mycoplasma pneumoniae positive control at 43K.

Lane 2: Legionella pneumophila positive control at 29K.

Lanes 3, 4, and 5: Mycoplasma pneumoniae culture-confirmed positive sputum specimens from atypical pneumonia patients.

Lanes 6, 7 and 8: Legionella pneumophila culture-confirmed positive sputum specimens from atypical pneumonia patients.

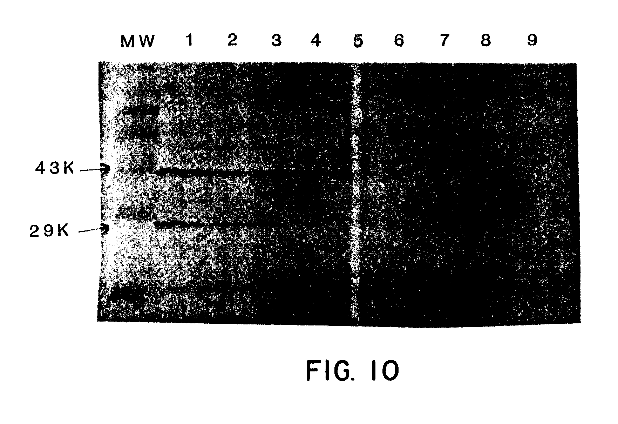

Fig. 10. Illustrates results showing the sensitivity of simultaneous monoclonal antibody immunoblot assay in detecting the M. pneumoniae polypeptide having an approximate molecular weight of 43K, and Legionella pneumophila polypeptide having an approximate molecular weight of 29K in spiked sputum specimens.

|  | M. pneumoniae | L. pneumophila |
|---|---|---|
| Lane 1: | 3200 ng/ml | 800 ng/ml |
| Lane 2: | 1600 ng/ml | 400 ng/ml |
| Lane 3: | 800 ng/ml | 200 ng/ml |
| Lane 4: | 400 ng/ml | 100 ng/ml |
| Lane 5: | 200 ng/ml | 50 ng/ml |
| Lane 6: | 100 ng/ml | 25 ng/ml |
| Lane 7: | 50 ng/ml | 12-5 ng/ml |
| Lane 8: | 25 ng/ml | 6-3 ng/ml |
| Lane 9: | Unspiked sputum |  |

Positive immunoblot bands were detected for the M. pneumoniae antigen having an approximate molecular weight of 43K in lanes 1 through 6, and for the L. pneumophila antigen having an approximate molecular weight of 29K in lanes 1 through 4. Sensitivity levels for both antigens were at 1OO ng/ml.


## EXAMPLES

Examples 1 and 2 below describe the preparation of a species-specific MAb of this invention, its characteristics, and its use in a Western immunoblot to diagnose M. pneumoniae infections.

Examples 3 and 4 describe the use of the species-specific MAb for Mycoplasma pneumoniae (M. pneumoniae) as well as MAbs specific for Legionella pneumophila and Chlamydia lipopolysaccharide, prepared according to the procedures used in Example 1, in the simultaneous assay technique of this invention; and exemplify the differential diagnosis procedure of this invention employing a panel of monoclonal antibodies in a Western immunoblot.

Reference strain microorganisms employed in the following examples were obtained from the American Type Culture Collection (ATCC) and the National Institutes of Health (NIH): Mycoplasma pneumoniae ATCC 15331; Mycoplasma hominis ATCC 23114; Mycoplasma faucium ATCC 25293; Mycoplasma lipophilum ATCC 271O4; Mycoplasma buccale NIH M714-O12-84; Mycoplasma fermentans NIH M713-OO2-84; Mycoplasma orale NIH M714-OO1-O84; Mycoplasma salivarium NIH M712-OO2-O84; Branhamella catarrhalis ATCC 8176; Candida albicans ATCC E-1O231; Escherichia coli ATCC 8739; Haemophilus influenzae ATCC 33391; Haemophilus parainfluenzae ATCC 33392; Neisseria siccaATCC 29256; Pseudomonas aeruginosa ATCC 9027; Staphylococcus aureus ATCC 25923; Staphylococcus aureus Cowan's strain ATCC 12598; Staphylococcus epidermidis ATCC 1499O; Streptococcus MG ATCC 9895; Streptococcus pneumoniae ATCC 334OO; Streptococcus pyogenes ATCC 12344; Streptococcus salivarius ATCC 13419. Klebsiella pneumoniae and Proteus mirabilis were obtained from University of California, Davis/Sacramento Medical Center and Streptococcus mutans from Hamilton Smith at Johns Hopkins University. Legionella pneumophilia serotype I was obtained from Paul Edelstein at the University of California, Los Angeles School of Medicine.

The present invention will be more readily understood by means of the following examples. However, these examples are set forth for the purpose of elaborating the different aspects of this invention and not intended to limit the invention in any way.

EXAMPLE 1

Preparation and Characterization of the 43K Species-Specific MAb for Mycoplasma pneumoniae

Antigen Preparations.

M. pneumoniae was grown in modified New York City medium broth (Granato et al., 1980. Am. J. Clin. Path. **73**:702-705) for 5-6 days using 32-oz glass prescription bottles. Cultures were harvested by scraping glass adherent organisms and washing them three times in phosphate-buffered saline (PBS) as described in Baseman et al., 1982. J. Bact. **151**:1514-1522. Other mycoplasma species were grown in PPLO glucose or PPLO arginine broths (Velleca et al., 1980, p. 113-119. Laboratory Diagnosis of Mycoplasma Infections, U.S. Department of Health and Human Services, Atlanta, GA.) and microorganisms were grown in tryptic soy broth (Difco) or on chocolate agar.

Polyclonal Antisera.

Rabbit anti-M. pneumoniae antiserum was obtained from Burroughs Wellcome. Mycoplasma species-specific antisera obtained from NIH were: Mule anti-M. pneumoniae M710-501-569; Mule anti-M. salivarium-M712-501-569; Mule anti-M. orale M-714-501-569; Mule anti-M. buccale M714-511-569; Mule anti-M. fermentans M713-501-569; Mule anti-M. hominis M711-501-560.

Immunizations.

Balb/c mice were initially immunized intraperitoneally (i.p.) with 100 $\mu$g of M. pneumoniae whole organism in complete Freund's adjuvant. They were subsequently boosted with 20 $\mu$g of antigen every two weeks, with the last injection given three days before fusion, for a total of five injections.

Fusion.

Balb/c spleen cells were fused with 6-thioguanine-resistant (HGPRT⁻) mouse myeloma cell lines Sp2/OAg14 (Shulman et al., 1978. Nature **276**: 269-270). The cells were fused according to a slightly modified procedure described in (Oi et al., 1980. Immunoglobulin-producing hybrid cell lines, p. 351-371. In B. Smichell and S. Schiigi (ed.), Selected methods in cellular immunology, W.J. Freeman Co., San Francisco). Fusion medium was 40% polyethylene glycol 1450 (J.T. Baker) in Dulbecco's Modified Eagle's Medium (GIBCO) with 15 mM Hepes. Cells were then plated at $5 \times 10^4$ cells/well in 96-well plates in Dulbecco's Modified Eagle's Medium 15 mM Hepes, containing 20% fetal calf serum, 10% NCTC medium (GIBCO), 2 mM glutamine, $1.1 \times 10^{-3}$ M cis-oxalacetic acid, $5.6 \times 10^{-4}$ M pyruvic acid, and 0.5 units/ml bovine insulin. Selection was applied 18 h after fusion by addition of $1 \times 10^{-4}$ M hypoxanthine and $1.1 \times 10^{-5}$ M azaserine (Foung et al., 1982. Proc. Natl. Acad. Sci. (USA) **79**:7484-7488). Ten to 21 days after the fusion, supernatants of grown hybrid cells were assayed for anti-M. pneumoniae antigen activity by enzyme immunoassay (EIA). Positive parental wells were cloned twice by limiting dilution.

EIA for Screening Hybridomas.

M. pneumoniae antigen was sonicated for 20 min, then centrifuged for 20 min in a microfuge. Sonicate supernatant was diluted in PBS to 1 $\mu$g Lowry protein/ml, and 50 $\mu$l was added to wells of polyvinylchloride plates for 60 min at 37°C. The material was aspirated and the wells were blocked with 1% bovine serum albumin (BSA) in PBS for 30 min at 37°C. After three washings with PBS + 0.1% Tween 20 (PBST), 50$\mu$l of undiluted cell culture supernatant fluid was added to wells and incubated for 60 min at 37°C. After three washings with PBST, 50 $\mu$l of horseradish peroxidase (HRP)-labeled goat antimouse IgG (Bio-Rad) diluted in 1% BSA + PBST was added, and wells were incubated for 60 min at 37°C. Unbound conjugate was washed four times in PBST then 100 µl of substrate buffer [3 mg/ml o-phenylenediamine•2HCl (Sigma) in 50 mM sodium citrate, 100 mM sodium phosphate, pH 5.5] was added to the wells. After 15 min at room temperature (RT), the reaction was stopped with 100 µl 1N HCl and absorbances were read in a spectrophotometer at 492 nm.

## Ascites Production.

Balb/C mice were injected i.p. with 0.5 ml pristane (Aldrich). After two weeks, the mice were injected with $5 \times 10^6$ of twice-subcloned hybridoma cells. Ascites fluid was harvested after 7 days.

## Gel Electrophoresis.

Sodium dodecyl sulfate-polyacrylamide gel electrophoresis (SDS-PAGE) employing 9% or 6-20% linear gradient acrylamide gels was performed by using the discontinuous SDS Tris glycine buffer system (Laemmli, V.K., 1970. Nature (London) **227**:680-685). Samples are brought to a concentration of 2% SDS, 0.1 M dithiothreitol (DTT), 10% glycerol in 0.1 M tris-HCl, pH 6.8 containing 0.01% bromophenol blue and heated for 5-10 minutes at 100°C. Silver staining of gels was performed as previously described (**Tsai et al.**, 1982. Anal. Biochem. **119**:115-119).

## Immunoblotting.

The electrophoretic transfer procedure described in **Burnette, W.N.**, 1981. A. Anal. Biochem. **112**:195-203 and **Towbin et al.**, 1979. Proc. Natl. Acad. Sci. (USA) **75**:4350-4354 was employed to transfer polypeptides from SDS-PAGE gels to nitrocellulose sheets. Transfers were performed in a Trans-Blot cell (Bio-Rad; Richmond, CA) for 60 min at 40V, 200 mA employing transfer buffer (25 mM Tris, 192 mM glycine, pH 8.8, 20% methanol). The nitrocellulose sheets were blocked for 30 min in a 1 M glycine solution containing 1% ovalbumin and 5% nonfat dry milk (18) at RT. After three washings in PBST, the sheets were probed with ascites fluid diluted 1:5,000 in wash solution + 0.1% nonfat dry milk and 0.1% ovalbumin for 60 min at 37°C. The sheets were washed three times in PBST and then incubated with HRP-labeled goat antimouse IgG for 30 min at RT. After four washes in PBST the sheets were exposed to the enzyme substrate solution consisting of 0.5 mg/ml 3,3'-diaminobenzidine•4HCl (DAB; Sigma), 0.02% NiCl$_2$, and 0.05% H$_2$O$_2$ in 10 mM Tris-HCl, pH 7.4 (**Hsu et al.**, 1982. J. Histochem. Cytochem. **30**:1079-1082). The reaction was stopped after 3-5 min by using distilled water.

## Immunostaining.

PBS-washed whole M. pneumoniae organisms were air dried on microscope slides and fixed for 20 min at RT with acetone, ethanol, or 1% glutaraldehyde in PBS. Ascites diluted in PBS containing 10% fetal bovine serum was applied to the slide and incubated for 30 min at 37°C. Slides were washed three times in PBS. Slides were subsequently incubated with HRP-labeled goat antimouse IgG for 15 min at 37°C, then washed three times. Slides were then incubated with DAB solution, as described above, and viewed under a microscope.

## Membrane Purification.

Membranes were purified as described in **Kahane et al.**, 1977. Infect. Immun. **18**:273-277. Briefly, glycerol-loaded organisms were lysed in distilled water. Membranes were washed four times by centrifugation and subsequently applied to a 30-54% (w/v) sucrose gradient and ultracentrifuged in a Beckman SW28 at 28,000 rpm for 3.5 h at 4°C.

Periodate and Protease Sensitivity.

The sensitivity of binding sites to periodate and protease treatment was determined as follows: 96-well polyvinylchloride plates were incubated with 1 μg/ml M. pneumoniae sonicate (50μl/well) for 2 h at RT. Plates were washed with PBST, then treated with either 0.05 M sodium-m-periodate in 0.05 M sodium acetate buffer, pH 8.8 (50 μl/well), overnight at 4°C or 100 mg/ml Proteinase K (Sigma Type XI) in PBS (50 μl/well) for 2 h at 37°C, as described in **Caldwell et al.,** 1984. Infect. Immun. 44:306-314. After the plates were washed 6 times with PBST, MAb binding was determined by the EIA described above using ascites fluid diluted 1:5000 in PBST instead of cell culture supernatant.

Mouse Immunoglobulin Isotype.

The immunoglobulin isotype of the MAb was determined by EIA. Rabbit antimouse IgG isotype-specific antisera IgG1, IgG2a, IgG2b, and IgG3 (Zymed Laboratories) followed by HRP-labeled goat antirabbit IgG (Cappel) were used to detect bound MAb. Incubations were for 60 min at RT.

Isolation of a hybridoma cell line producing anti-M. pneumoniae MAb.

Hybridoma cell culture supernatants positive by EIA were further screened by immunoblot assay. One MAb-secreting hybridoma designated MP16C9 was identified that bound to a polypeptide with an apparent molecular weight of 43,000 (43K). EIA analysis of MP16C9 indicated it was an IgG1 isotype. This MAb also bound to a 43K polypeptide in a purified membrane preparation as shown in Fig. 1. This 43K polypeptide appeared to correspond to the 43K polypeptide that strongly bound the rabbit polyclonal antibody. The MP16C9 MAb binding site appeared to be destroyed in EIA by protease digestion but not by periodate oxidation treatment.

Anti-43K MAb reactivity with other mycoplasma.

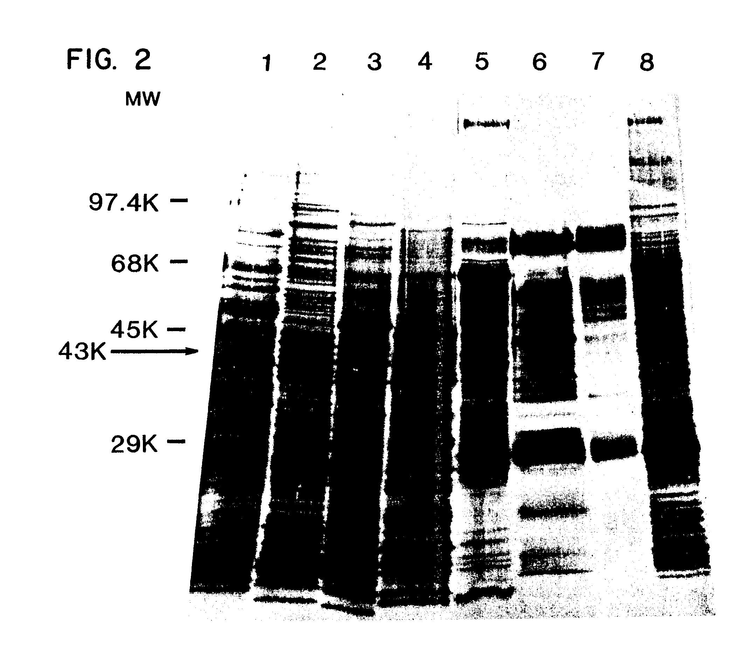

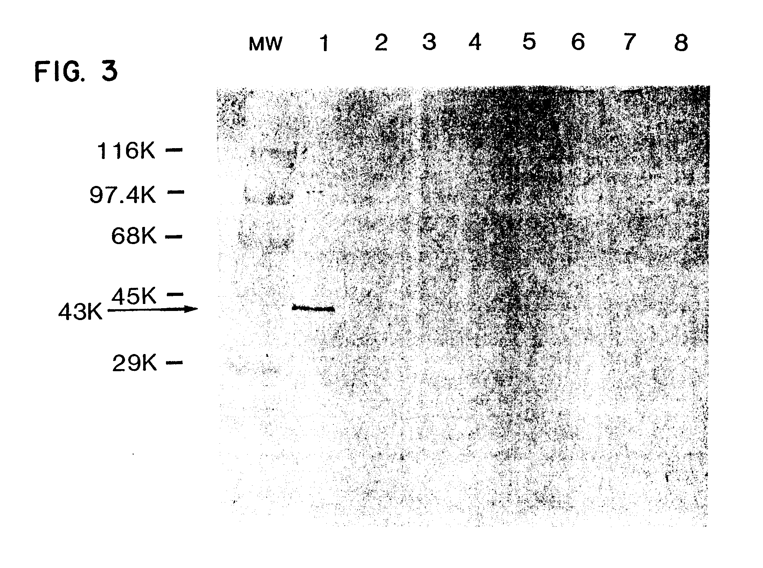

Although 43K polypeptides were observed in the normal mycoplasma species shown by silver staining patterns in Fig. 2, no MAb binding in the immunoblot assay occurred to any of these polypeptides from these organisms as shown in Fig. 3. This MP16C9 MAb was also tested by EIA (Table 1) for reactivity to M. fermentans, M. hominis, M. salivarium, M. orale, M. buccale, M. lipophilum, and M. faucium. No reactivity was observed by EIA to any of these mycoplasma species.

Reactivity with M. pneumoniae clinical isolates.

MP16C9 MAb was evaluated by immunoblot for reactivity with 18 M. pneumoniae clinical isolates. The 43K polypeptide in all 18 isolates was found to be reactive with MP16C9 MAb.

Reactivity with normal flora bacteria.

Representative organisms potentially found in the normal or diseased human respiratory tract were tested by immunoblot for reactivity to the MP16C9 MAb as shown in Table 2. None of the organisms tested were found to contain polypeptides that bound the MP16C9 MAb.

Immunostaining Characteristics.

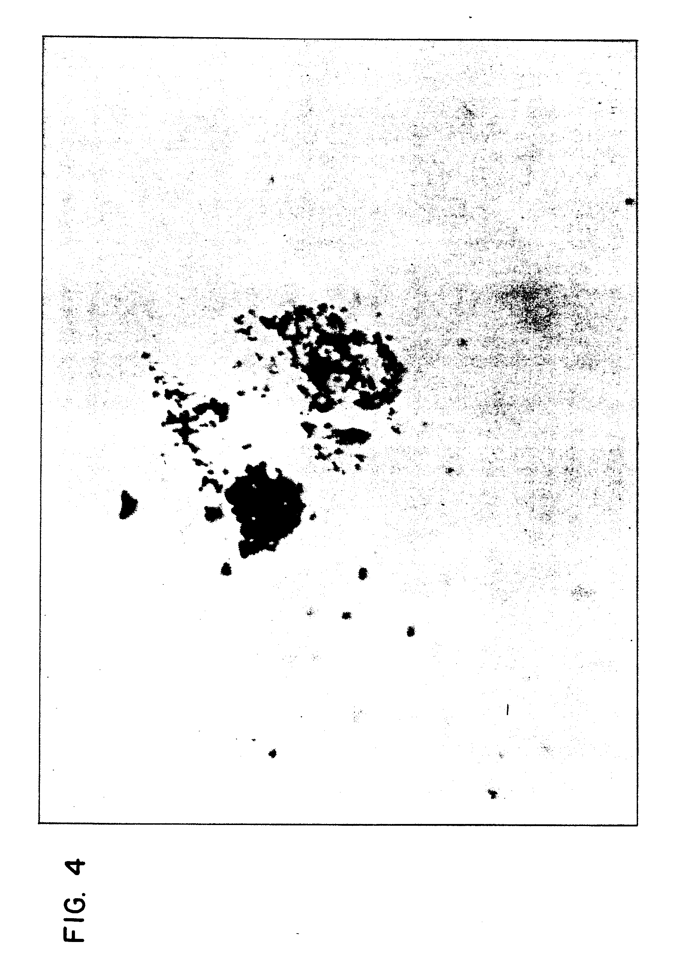

Whole organisms fixed with ethanol or acetone bound the MP16C9 MAb as shown in Fig. 4. Organisms fixed with glutaraldehyde did not.

## Table 1. EIA Reactivities of Human Mycoplasmatales to Species-Specific Polyclonal Antisera and MP16C9 Monoclonal Antibodies

| Antigen | $\Delta OD_{492}$[1] | |
| --- | --- | --- |
| | Monoclonal MP16C9 Anti-M. pneumoniae | Polyclonal Anti-Mycoplasma Species[2] |
| Growth medium | 0.04 | 0.44 |
| M. pneumoniae | 0.96 | 1.38 |
| M. salivarium | 0.02 | 1.19 |
| M. orale | 0.07 | 1.10 |
| M. buccale | 0.05 | 0.58 |
| M. fermentans | 0.05 | 1.15 |
| M. lipophilum | 0.12 | ND[3] |
| M. hominis | 0.10 | 0.55 |
| M. faucium | 0.08 | ND |

[1] Difference in absorbance between antigen-coated well and PBS control well. EIA was performed as described in the text of the specification.

[2] NIH species-specific antisera (see test) was used as primary antibody. HRP-labeled rabbit antihorse IgG (Miles) was used as second antibody.

[3] Not done.

### Table 2.  Reactivity of Microorganisms with
### Monoclonal Antibody MP16C9

Microorganism MAb-Immunoblot Reactivity[*]

| | |
|---|---|
| Escherichia coli | (-) |
| Klebsiella pneumoniae | (-) |
| Proteus mirabilis | (-) |
| Pseudomonas aeruginosa | (-) |
| Staphylococcus aureus | (-) |
| Staphylococcus aureus (Cowan's strain) | (-) |
| Staphylococcus epidermidis | (-) |
| Streptococcus mutans | (-) |
| Streptococcus salivarius | (-) |
| Streptococcus pneumoniae | (-) |
| Streptococcus MG | (-) |
| Streptococcus pyogenes | (-) |
| Branhamella catarrhalis | (-) |
| Haemophilus influenzae | (-) |
| Neisseria sicca | (-) |
| Haemophilus parainfluenzae | (-) |
| Chlamydia trachomatis | (-) |
| Candida albicans | (-) |
| Legionella pneumophila type 1 | (-) |

* Approximately 100 ng of PBS-washed microorganism was loaded per lane and run in 9% SDS-PAGE.  Subsequent electrophoretic transfers onto nitrocellulose sheets were probed with MP16C9 MAb as described in the text.  Negative reactivity was defined as absence of MAb binding to polypeptides in the molecular weight ranges of 30,000 to 60,000.

EXAMPLE 2

Use of Species-Specific MAb in Western Immunoblot Assay

Mycoplasma pneumoniae

(ATCC 15531) was grown for 4-6 days in 32-oz prescription bottles using modified New York City medium (Granato et al., 1983. J. Clin. Micro. 17:1077-1080). Glass adherent colonies were scraped off and harvested as previously described. Quantitation of infectivity was done by color change assay as described in Velleca et al., supra, p. 105.

Throat Swabs

Pharyngeal swabs were obtained from students visiting the Syracuse University Student Health Center during December 1980 to April 1981. These patients were symptomatic for respiratory infection with chief complaints of sore throat, nasal congestion or cough. Swabs were expressed phosphate-buffered saline (PBS) containing Tween 20 and stored frozen at -70°C.

Sputum Specimens

Sputum samples were obtained from young adults diagnosed as having atypical pneumonia. Serum cold-agglutinin titers were ≥ 1:128. Specimens were cultured for the presence M. pneumoniae as previously described in Granato et al., supra.

Monoclonal Antibody .

Ascites purified MAb to a M. pneumoniae 43,000 molecular weight polypeptide was prepared as described above in Example 1.

MAb Immunoblot Assay

Clinical specimens were first subjected to sodium dodecyl sulfate polyacrylamide gel electrophorsis (SDS-PAGE) using the discontinous SDS tris-glycine buffer system [Laemmli, V.K., 1970. Nature (London) 227:680-685]. Gels consisted of a 9% acrylamide separating gel and a 6% acrylamide stacking gel cast in a 1.5 $^\times$ 80 $^\times$ 100 mm "minigel". Clinical specimens were boiled for 5 minutes (Sputums 10 minutes) in equal volumes of specimen treatment buffer (0.2 M Tris-HCl, pH 6.8, 4% (w/v) SDS, 0.2 M dithiothreitol, 20% (v/v) glycerol, 0.02% bromophenol blue). 30 $\mu$l was loaded per lane and electrophoresis was conducted for 60 min at 50 mA constant current. Prestained protein molecular weight markers (Bethesda Research Laboratories) were included to chart the progress of the electrophoretic separation. The separated polypeptides were electrophoresed onto nitrocellulose (Schleicher and Schuell) employing procedures described in Burnette, supra; Towbin, Proc. Nat. Acad. Sci. (1979), supra. Transfers were performed in a Trans-blot cell (Bio-Rad; Richmond, CA) for 45 min at 40V, 200mA using a transfer buffer comprised of 25 mM Tris-HCl, 192 mM glycine and 20% (v/v) methanol. The nitrocellulose (NC) was blocked for 15 min in 1 M glycine, 5% nonfat dry milk, 1% ovalbumin. This blocking reagent is a modification of an immunoblot blocking solution described in Johnson et al., 1984. Gene Anal. Techn. 1:3-8. After a 5 min wash in PBS + 0.1% Tween 20 (PBST) the NC sheet was probed with MAb diluted in PBST + 20% fetal bovine serum (FPBS) for 60 min at 37°C. NC was washed three times for a total of 10 min then incubated with biotinylated horse antimouse IgG (Vector Laboratories) in FPBS for 30 min at room temperature (RT). After washing, the NC was exposed to avidin-biotin-horseradish peroxidase reagent (Vector Laboratories) for 15 min at RT. The NC was then rinsed quickly twice with PBST, then washed twice with fixing buffer (1% dextran sulfate in 0.1 M sodium fumarate containing 1 mM EDTA, pH 4.8). Fixing buffer was decanted and enzyme substrate solution (0.1 mg/mL tetramethylbenzidine, 0.02% $H_2O_2$ in 0.1 M sodium fumarate, 1 mM EDTA pH 4.8) was added. Color development was allowed to proceed for 15 min or until purple colored bands appeared. The reaction was stopped by rinsing in distilled water.

MAb Immunoblot Assay--Results.

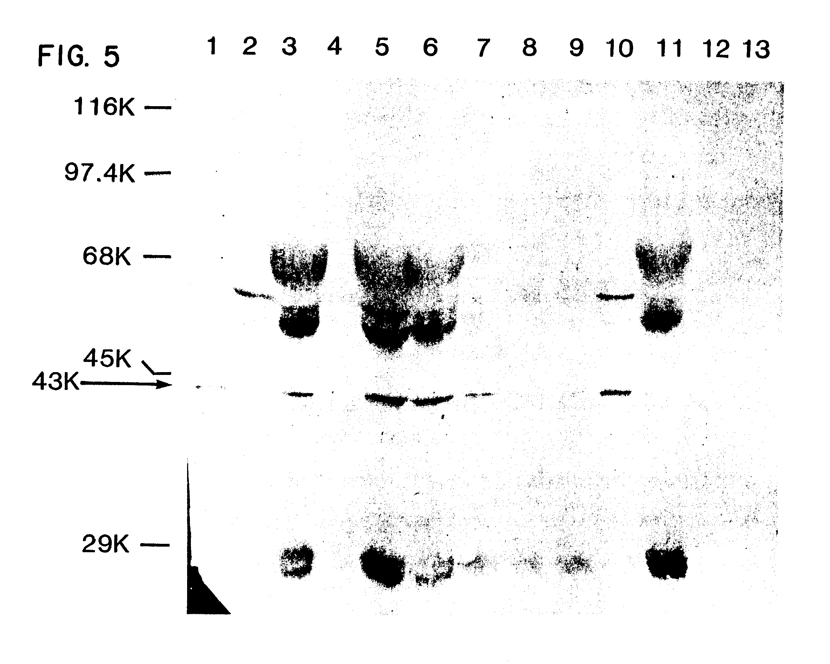

The initial evaluation of sputum specimens was conducted on 14 cm $\times$ 14 cm gels as shown in Fig. 5. Specific MAb binding to the 43K polypeptide could be well differentiated from any nonspecific MAb bound to immunoglobulin heavy and light chains. We subsequently developed an immunoblot procedure that could be completed within 5 hr using a minigel electrophoresis apparatus. An outline of the time frame for completing this assay is outlined in Table 3.

MAb Immunoblot Assay Sensitivity.

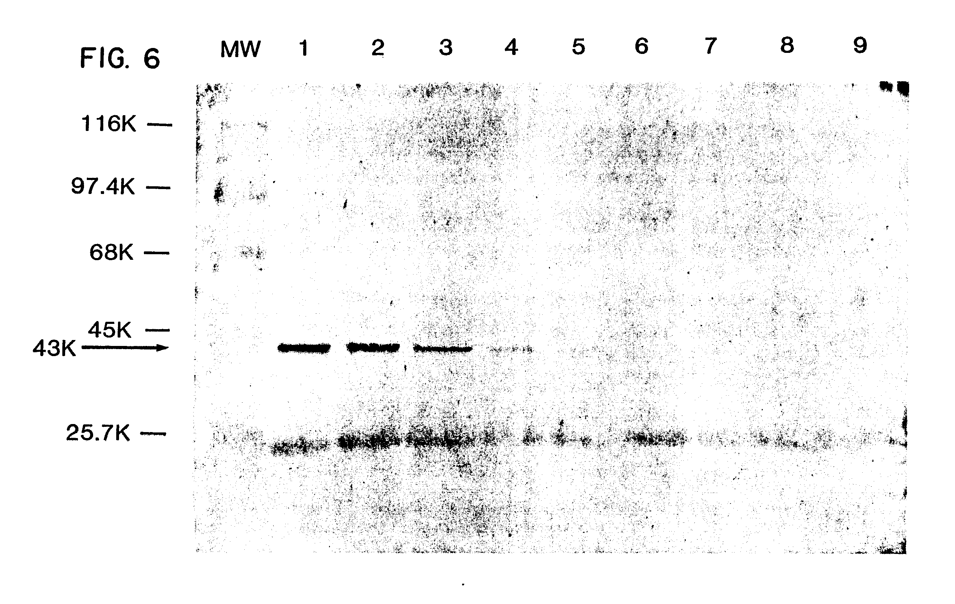

The sensitivity of this assay was determined by spiking normal sputum with serially diluted antigen. The M. pneumoniae stock used contained 3.2 mg/mL of Lowry protein and a titer of 5 $\times$ 10$^7$ CCU/mL. 43K polypeptide antigen was detectable in as little as 10 ng/lane of Lowry protein whole organism as shown in Fig. 6. This amount of organisms corresponds to a concentration of 200 ng/mL of specimen. Our estimates indicate that this amount correlates to a sensitivity level of 3200 CCU/mL.

MAb Immunoblot Assay of Atypical Pneumonia Sputum Specimens.

The MAb immunoblot assay detected 43K polypeptide in 10 of 24 atypical pneumonia sputum samples as illustrated in Fig. 7. M. pneumoniae culture confirmation of these specimens was not possible due to overgrowth of normal flora bacteria. It also detected the 43K polypeptide in each of three sputums tested where culture confirmation of M. pneumoniae was obtained. No 43K polypeptide could be detected from ten sputum samples from normal individuals. In addition, three sputum specimens culture-confirmed for Legionella pneumophila were tested and found to be negative for the 43K polypeptide.

MAb Immunoblot Assay of Throat Swab Specimens

The MAb immunoblot assay detected 43K polypeptide in 29 of 30 throat swabs specimens which had been confirmed culture positive for M. pneumoniae. These results are illustrated in Fig. 8. Less nonspecific staining of immunoglobulin heavy and light chains was observed with these specimens compared with sputum specimens. No antigen was detected in twenty-five throat swabs from normal individuals. A summary of the specimens tested with the MAb immunoblot assay is shown in Table 4.

## Table 3. MAb Immunoblot Assay Protocol and Time Flow[1] with Minigel

| STEP | TIME |
|---|---|
| 1. Solubilize Sample/Load Gel | 15 min |
| 2. Electrophoresis | 60 min |
| 3. Transfer | 45 min |
| 4. Block | 15 min |
| 5. Wash | 5 min |
| 6. Probe Primary MAb | 60 min @ 37°C |
| 7. Wash | 10 min |
| 8. Biotinylated Second Antibody | 30 min |
| 9. Wash | 10 min |
| 10. Avidin-HRP | 15 min |
| 11. Wash/Fix | 10 min |
| 12. TMB | 15 min |
| | 290 min    4.8 hr |

[1] Assumes use of precast gels.

## Table 4. Summary of Sputum Specimen Testing
## By 43K MAb Immunoblot Assay

| Patient Group | Specimen | Number positive for 43K by MAb Immunoblot Assay |
|---|---|---|
| Atypical pneumonia culture confirmation for M. pneumoniae not obtainable | Sputum | 12/20 |
| Atypical pneumonia culture positive for M. pneumoniae | Sputum | 3/3 |
| Atypical pneumonia culture positive for Legionella | Sputum | 0/3 |
| Normal | Sputum | 0/10 |
| Pharyngitis, culture positive for M. pneumonaie | Throat swabs | 29/30 |
| Normal | Throat swabs | 0/20 |

EXAMPLE 3

Differential Diagnosis of Mycoplasma pneumonia Versus Legionella pneumophila

Pneumonia is considered to be the leading cause of death from infectious disease in the United States (ref. 4). Empirical therapy for these infections must often be initiated prior to specific etiologic agent identification because of difficulties in rapid diagnosis. These difficulties include lack of sensitivity of Gram stained sputum, time delay for culture results and difficulties in the growth of agents.

This is clearly the case in pneumonias caused by Mycoplasma pneumoniae, and Legionella pneumophila. That there are no unique clinical or radiographic features of these disease agents (ref. 1), diagnosis is often entirely dependent on serology or isolation which requires specialized experience and media (refs. 2, 12). The development of an assay to directly detect the presence of said M. pneumoniae specific antigen in sputum specimens through the use of a monoclonal antibody in an immunoblot procedure is described above. Because of spatial separation of antigens achieved by electrophoresis in this assay, it has the potential to be employed for the simultaneous detection of antigens of different molecular weights from different disease agents. Once separated by electrophoresis and subsequently transferred to nitrocellulose by electroelution, these different antigens can be detected with monoclonal antibodies specific to their respective antigens.

By employing two species-specific monoclonal antibodies, one that binds to said 43K molecular weight polypeptide of M. pneumoniae and a second that binds to a 29K molecular weight polypeptide of L. pneumophila, an immunoblot assay can simultaneously detect these two antigens directly in clinical specimens. The characteristics and sensitivity of this assay system are herein described.

For the experiments described in this example, the monoclonal antibodies specific for the 43K M. pneumoniae polypeptide are prepared according to the procedures of Example 1.

Similarly, a monoclonal antibody against a 29K Legionella antigen can be prepared according to procedures analogous to those described in Example 1. Legionella was grown on enriched Legionella agar (Difco). Cultures were washed three times in PBS. An approximately 29K molecular weight polypeptide of Legionella pneumophila, a species specific antigen, is described in Gosting et al., J. Clin. Microbiol., Vol. 20, No. 6:1031-2035 (Dec., 1984) (ref. 20).

Alternatively, monoclonal antibodies to such 29K antigen are also available commercially from an immunofluorescent Legionella antibody test kit (product 10.149-0040) from Genetic Systems (Seattle, WA). Further, alternatively, a monoclonal antibody to an approximate 29K molecular weight polypeptide of Legionella pneumophila as described in Para et al. (1985 Abstracts of the 1985 Interscience Conference on Antimicrobial Agents and Chemotherapy #1079, p. 291) could be employed in this example. Legionella pneumophila serotype I was obtained form Paul Edelstein at UCLA School of Medicine.

A modification of the immunoblot procedure as described above in Example 1 was employed. Instead of the HRP-labelled anti-mouse IgG conjugate employed in Example 1, monoclonal antibody-probed nitrocellulose was incubated with biotinylated anti-mouse IgG for 30 minutes at room temperature (RT), followed by washing. Avidin-biotin-HRP complex was then added for 15 minutes at RT.

## Clinical Specimens.

Sputum samples were obtained from patients exhibiting atypical pneumonia. These specimens were cultured for Mycoplasma pneumoniae or Legionella pneumophila and positive colonies were identified by standard techniques.

## Assay Sensitivity.

The steps in the procedure are illustrated in Table 5. The assay can be performed in less than 5 hours when using precast minigels as herein exemplified. Sensitivity was determined by spiking whole organisms of L. pneumophila and M. pneumoniae into sputum from a normal individual. Specific 43K and 29K antigens could be detected at levels of 100 ng/ml of both organisms as shown in Fig. 10. This corresponded to $10^6$ CFU/ml of Legionella and 3200 CCU/ml of M. pneumoniae.

## Clinical Specimens.

The results of testing sputum specimens are shown in Fig. 9. The M. pneumoniae 43K antigen was detected in all three sputum specimens culture-confirmed for M. pneumoniae. The L. pneumophila 29K antigen was also detected in three of three culture-confirmed sputums from Legionella patients.

This assay clearly distinguishes between M. pneumoniae and L. pneumophila specific antigens in the number of specimens tested herein. Immunofluorescent (ref. 5), counter-immunoelectrophoretic (ref. 13) and latex agglutination assays (refs. 6, 10) have been previously employed to detect antigens of different etiologic agents in specimens from pneumonia patients. These assay systems have had difficulty in technically addressing the problems of liquification of the sample and solubilization of the antigen. Latex agglutination assays, in general, have also encountered nonspecific false positive reactions caused by the adhesive nature of the material of this type of specimen.

The simultaneous monoclonal antibody immunoblot assay described herein is very capable of directly addressing these difficulties. The use of SDS and DTT treatment coupled with heating prior to electrophoresis, liquifies the sample and solubilizes both the M. pneumoniae and L. pneumophila polypeptides. This method also spatially separates the 43K and 29K polypeptides from any specimen proteins potentially causing nonspecific false positive reactions. This assay, with the use of commercially available precast gels, is not much more involved than a standard enzyme immunoassay. It definitely provides an alternative assay method for samples that cannot be tested by conventional culture due to the overgrowth of normal flora organisms. As shown in Table 5, it can be performed in less than five hours when using precast minigels.

This format also allows the addition of monoclonal antibodies to other etiologic agents, provided that their reactive polypeptides can be spatially distinguished from the 29K and 43K polypeptides. Selecting a gradient gel appropriate for such separation is desirable. Haemophilus influenzae, Streptococcus pneumoniae, Chlamydia and influenza virus can be differentially detected with this assay. It would be necessary to prepare monoclonal antibodies as indicated above to polypeptides differing in molecular weights which are diagnostic for each such microorganisms. Below in Example 4, a 5K genus-specific lipopolysaccharide diagnostic for Chlamydia (ref. 19) is employed in a simultaneous immunoassay.

### Table 5. Monoclonal Immunoblot Assay Protocol and Time Flow[1] Employing Minigel

| STEP | TIME |
|------|------|
| 1. Solubilize Sample/Load Gel | 15 min |
| 2. Electrophoresis | 60 min |
| 3. Transfer | 45 min |
| 4. Block | 15 min |
| 5. Wash | 5 min |
| 6. Probe Primary MAb | 60 min @ 37°C |
| 7. Wash | 10 min |
| 8. Biotinylated Second Antibody | 30 min |
| 9. Wash | 10 min |
| 10. Avidin-Biotin-HRP | 15 min |
| 11. Wash | 10 min |
| 12. DAB | 5 min |
| | 280 min    4.7 hr |

[1] Assumes use of precast minigels.

EXAMPLE 4

Differential Diagnosis of M. pneumoniae, L. pneumophila and Chlamydia

A. Preparation of Monoclonal Antibodies

The monoclonal antibodies against M. pneumoniae and L. pneumophila are prepared or are available as indicated in Example 3. Further, for this example, a monoclonal antibody is prepared analogously to the method of Example 1 for an approximately 5K molecular weight genus-specific Chlamydia lipopolysaccharide antigen (that is for the species C. trachomatis and C. psittaci) as described in Caldwell et al., 1984, Inf. & Immun., Vol. 44, No. 2:306-314 (ref. 19).

B. Simultaneous Monoclonal Antibody Immunoblot Assay for M. Pneumoniae. L. Pneumophila, and Chlamydia psittaci.

Immunoblots (Westerns) can be performed by a modification of the procedure of Burnette, et al., Anal. Biochem. 112: 195 (1981). Sputum specimens and throat swabs as well as Legionella, Mycoplasma, and Chlamydia positive control samples are brought to a concentration of 2% SDS, 0.1 M DTT, 10% glycerol and 0.1 M tris-HCL, pH 6.8 with 0.01 bromphenol blue and heated at 100° C for 5 to 10 minutes to completely liquify the sample.

Legionella, Chlamydia and Mycoplasma positive control materials are prepared by suspending $10^6$ CFU of each organism per ml of PBS. Chlamydia positive control material is prepared by suspending $10^6$ PFU/ml of PBS.

After the heat treatment, these samples are applied to a 10 to 16% gradient polyacrylamide 14 × 14 cm slab gel, 0.1% SDS, 0.1 M tris-HCL, pH 8.85 that had a 4% stacking gel in 0.1% SDS and 0.1 M tris-HCL pH 6.8. and electrophoresed at a constant current of 25mA (Laemmli. U.K , Nature, 227:680 ,1970).

Antigens are transferred to a 0.45 micron nitrocellulose sheet in 20% methanol, 0.025 M tris-HCl, 0.192 M glycine, pH 9.0 buffer under constant voltage of 40 volts for 1 hr. Nitrocellulose is blocked with 1 M glycine, 1% ovalbumin and 5% dry milk for 15 min at RT. After rinsing the sheets with PBS containing 0.1% tween 20, sheets are probed for one hour at 37° C with mixture of monoclonal antibodies to the different disease agents.

This mixture contains a species-specific anti-M. pneumoniae monoclonal antibody which binds to a 43K molecular weight polypeptide, as indicated above, a species-specific anti-L. pneumophila monoclonal antibody which binds to a 29K molecular weight polypeptide (Gosting, J. of Clin. Micro. 20:1031, 1984) (ref. 20), and a genus-specific anti-Chlamydia monoclonal antibody which binds to a 5K molecular weight lipopolysaccharide (Caldwell, et al, Infect. & Immun.44:306, 1984) (ref. 19).

After rinsing the nitrocellulose sheet in PBS plus 0.1% tween 20 (PBST), bound monoclonal antibody is detected by biotinylated anti-mouse IgG antibodies diluted in 10% fetal calf serum in PBS for thirty minutes at RT. After washing in PBST, the nitrocellulose is incubated with the avidin-biotin-HRP complex for 15 minutes at RT.

After rinsing the nitrocellulose sheet in PBST, bound enzyme conjugate is detected by incubating a solution containing diaminobenzidine and hydrogen peroxide in tris-HCl at pH 8.0.

In the lane containing the M. pneumoniae postive control a dark band will appear at an approximate molecular weight of 43,000. In the lane containing the L. pneumophila positive control a dark band will appear at an approximate molecular weight of 29,000. In the lane containing the Chlamydia positive control a dark band appears at an approximate molecular weight of 5,000 which may appear in the dye front.

In the lanes containing patient specimens, if a dark band appears at a molecular weight of 43,000 corresponding to the M. pneumoniae positive control then M. pneumoniae is present in that sample. If a dark band appears at a molecular weight of 29,000 corresponding to the L. pneumophila positive control, then that sample contains L. pneumophila. If a dark band appears at molecular weight of 5,000 corresponding to the Chlamydia positive control then Chlamydia is present in that sample.

Some specimens may demonstrate dark bands corresponding to molecular weights other than 29,000 , 43,000, or 5,000. These are interpreted as nonspecific reactions and considered negative.

References Cited

1. Dean, 1981. Mycoplasmal Pneumonias in the Community Hospital. Clinics in Chest Medicine 2: 121-131.

2. Edelstein et al., 1985. Clinical Utility of a Monoclonal Direct Fluorescent Reagent Specific for Legionella pneumophila: Comparative Study With Other Reagents. J. Clin. Micro. 22: 419-421.

3. Erlich et al., 1979. Filter Affinity Transfer: A new technique for the in situidentification of proteins in gels. J. Biol. Chem 254: 12,240-12,247.

4. Finegold et al., 1985. Lower Respiratory Tract Infection. Amer. J. Med. 79(S5B):73-77.

5. Hers, 1963. Fluorescent Antibody Technique in Respiratory Disease. Am. Rev. Resp. Dis. 88:316-333.

6. Leinmen et al., 1978. Comparison of counter-current immunoelectrophoresis, latex agglutination and radioimmunoassay in detection of soluble capsular polysaccharide antigens of Haemophilus influenzae type b and Neisseria meningitis of group A or C.J. Clin. Path. 31:1172-1176.

7. Drouillard et al., Detection of Additional Proteins Implicated in Virulence and Hemadsorption of Mycoplasma pneumoniae. Abstracts for 1985 Annual Meeting of the American Society for Microbiology Abst. G20.

8. Kahane et al., Dec. 1985. Detection of the Major Adhesin P1 in Triton Shells of Virulent Mycoplasma pneumoniae. Inf. & Immun. 50 (No. 3):944-946.

9. McKeller, 1985. Treatment of Community-Acquired Pneumonias. Am. J. Med. 79 (Suppl 2A):25-31.

10. Thirumourthi et al., 1979. J. Clin. Microbiol. 9:28-32.

11. Towbin et al., 1979. Proc. Nat. Acad. Sci. U.S.A. 76:4350-4354.

12. Tully et al., 1979. Enhanced Isolation of Mycoplasma pneumoniae from throat washings with a newly modified culture medium. J. Inf. Dis. 139 :478-482.

13. Wiernik et al., 1978. The Value of Immunoelectroosmophoresis (IEOP) for Etiological Diagnosis of Acute Respiratory Tract Infections due to Pneumococci and Mycoplasma pneumoniae Scand. J. Infect. Dis. 10:173-176.

14. Towbin et al., U.S. Patent No. 4,452,901, filed April 20, 1981; issued June 5, 1984.

15. Bittner et al., 1980. Electrophoretic Transfer of Proteins and Nucleic Acids from Slab Gels to Diazobenzyloxymethyl Cellulose or Nitrocellulose Sheets. Anal. Biochem. 102:459-471.

16. Towbin et al., 1984. Immunoblotting and dot immunoblotting--current status and outlook. J. Immunol. Methods. 72 (No. 2):313-340.

17. McMichael et al., 1981. J. Immunol. Methods. 45:79-94.

18. Gallo et al., U.S. Patent No. 4,520,113, filed April 23, 1984; issued May 28, 1985.

19. Caldwell et al., May, 1984. Monoclonal Antibody Against a Genus-Specific Antigen of Chlamydia Species: Location of the Epitope on Chlamydial Lipopolysaccharide. Inf. & Immun. 44 (No. 2):306-314.

20. Gosting et al., December, 1984. Identification of a Species-Specific Antigen in Legionella pneumophila by a Monoclonal Antibody. J. Clin. Microbiol. 20 (No. 6):1031-1035.

21. Kuriyama et al., 1982. JNCI. 68:99-205.

22. Mitsuma et al., 1972. Biochem. Biophys. Res. Commun. 46:2107-2113.

23. Ljunggren et al., 1976. Acta Endocrinol. 81:487-494.

24. Haynes et al., 1977. Ann. Clin. Biochem. 14:12-15.

25. Vihko et al., 1981. Multicomponent Radioimmunoassay: Simultaneous Measurement of Choriomammotropin and Pregnancy-Specific $\beta_1$-Glycoprotein in Pregnancy Serum. Clin. Chem. 27:1744-1746.

26. Fridlender et al., U.S. Patent No. 4,315,907, Combined Heterogeneous Specific Binding Assay, filed Oct. 29, 1979, issued Feb. 16, 1979.

27. Gehle et al., J. Immunol. Methods, 26:381-389 (1979).

28. Blake et al., 1982. Clin. Chem. 28:1469-1473.

Conclusion

This invention provides a rapid diagnostic test that is currently needed by clinicians for making a differential diagnosis or simultaneously detecting a number of etiologic agents. The long waits and possible inaccuracies which are now inherent in the diagnostic methods currently used in clinical laboratories are eliminated by the diagnostic methods of this invention.

It may be seen, further, that the invention provides a method of diagnosing M. pneumoniae infections. The invention provides a rapid diagnostic test that is currently needed by clinicians.

Various modifications of the invention in addition to those shown and described herein will become apparent to those skilled in the art from the foregoing description. Such modifications are intended to fall within the scope of the appended claims.

**Claims**

1. A species-specific monoclonal antibody which binds to an epitope on a Mycoplasma pneumoniae membrane polypeptide with a molecular weight of approximately 43 kilodaltons.

2. A monoclonal antibody as defined in claim 1 which is designated MP16C9, and functional equivalents thereof.

3. A hybridoma, and progeny thereof, which secretes monoclonal antibodies as defined in claim 1.

4. A monoclonal antibody as defined in claim 1 which reacts with M. penumoniae but does not react with normal flora mycoplasma species.

5. A monoclonal antibody as defined in claim 1 or a functional equivalent thereof which can be used in a Western immunoblot assay to diagnose M. pneumoniaeinfections.

6. A method of detecting a defined, disease-related antigen in a clinical specimen by employing a monoclonal antibody which binds to said antigen in a Western immunoblot assay.

7. A diagnostic method as defined in claim 6 wherein the antigen is a M. pneumoniae membrane polypeptide with an approximate molecular weight of 43 kilodaltons.

8. A diagnostic test kit for detecting M. pneumoniae infections using the Western immunoblot technique comprising the monoclonal antibody of claim 1.

9. A diagnostic kit as defined in claim 8 further comprising an indicator system to detect bound monoclonal antibody.

10. A method of detecting simultaneously a variety of antigens in a single Western immunoblot assay by monoclonal antibodies specific to each antigen to be detected.

11. A method according to claim 10 wherein 1 to 10 antigens are targets of the immunoassay.

12. A method according to claim 10 wherein one of the monoclonal antibodies employed is murine IgG1 monoclonal antibody which binds to a M. pneumoniaemembrane polypeptide having an approximate molecular weight of 43K.

13. A method according to claim 12 wherein another monoclonal antibody employed is one that binds to a species-specific antigen of Legionella pneumophila which has an approximate molecular weight of 29K.

14. A method according to claim 13 wherein a further monoclonal antibody employed is one that binds to a genus-specific lipopolysaccharide of Chlamydia trachomatis or psittaci which has an approximate molecular weight of 5K.

15. A differential diagnostic method comprising the steps of

a. selecting an appropriate panel of monoclonal antibodies, each of which is diagnostic for an etiologic agent of clinical symptoms displayed by the patient under diagnosis;

b. employing said monoclonal antibodies in a Western immunoblot assay to test one or more clinical specimens from said patient; and

c. diagnosing the patient as having one or more of the etiologic agents or eliminating as a causative agent one or more of the etiologic agents tested for by said immunoblot by examining the appearance or non-appearance of specific bands for correspondence to the appropriate molecular weights of the target antigens from said etiologic agents in the test results.

16. A diagnostic kit comprising a panel of monoclonal antibodies each of which binds to an etiologic agent that causes symptomatically related diseases.

FIG. 1

FIG. 2

FIG. 3

MW   1   2   3   4   5   6   7   8

116K —

97.4K —

68K —

43K $\frac{45K}{\longrightarrow}$ —

29K —

FIG. 4

FIG. 5

FIG. 6

MW    1    2    3    4    5    6    7    8    9

116K —

97.4K —

68K —

45K —
43K →

25.7K —

0 254 384

FIG. 7

116K —
97.4K —
68K —
45K —
43K —
29K —

0 254 384

FIG. 8

FIG. 9

FIG. 10